# EUROPEAN PATENT SPECIFICATION

(11) **EP 1 947 075 B1**
(45) Date of publication and mention of the grant of the patent: **10.11.2010**
(21) Application number: 07001109.3
(22) Date of filing: 19.01.2007
(51) Int. Cl.: C07C 2/30, C07C 11/02

(54) **Method for preparation of linear alpha-olefins and reactor system therefore**
Herstellungsverfahren für lineare Alpha-Olefine und Reaktorsystem dafür
Procédé de préparation d'alpha-oléfines linéaires et système de réaction correspondant

(43) Date of publication of application: 23.07.2008
(73) Proprietor: Saudi Basic Industries Corporation, Riyadh 11422 (SA); Linde AG, 80331 München (DE)
(72) Inventor: Fritz, Peter M., 82008 München (DE); Bölt, Heinz, 82515 Wolfratshausen (DE); Winkler, Florian, 80469 München (DE); Müller, Wolfgang, 81245 München (DE); Schneider, Richard, 82449 Uffing (DE); Wellenhofer, Anton, 81247 München (DE); Mousa, Fuad, 11551 Riyadh (SA)
(74) Representative: Winkler, Andreas Fritz Ernst

(56) References cited:
- WO-A-2004/029011
- DE-C1- 4 338 414
- US-A- 3 051 557
- US-A- 5 523 508

## Description

The present invention relates to a method for the preparation of linear alpha-olefins by oligomerization of ethylene in a reactor in the presence of a catalyst and solvent and a reactor system therefore.

Methods for the preparation of linear alpha-olefins are widely known in the art (US-A-5 523 508). The methods are carried out in a reactor, usually in the presence of a catalyst, wherein an outlet stream is discharged from the reactor comprising solvent, catalyst and linear alpha-olefins. As is common knowledge, the linear alpha-olefins may comprise olefins having short chains, i.e. having 4 to 18 carbon atoms, which are liquid under oligomerization conditions, but may also comprise olefins having 20 or more carbon atoms, which are usually solid or semi-solid under oligomerization conditions, especially after discharge from the reactor for transferring the outlet stream to a catalyst removal section for deactivating and removing the catalyst.

Especially under start-up conditions and at extremely low ambient temperatures, thus, a deposition of heavy molecular weight oligomers, especially in the catalyst removal section, can take place. The consequence is plugging of equipment and piping systems.

Additionally, it is known that for some prior art methods cooling and solidification of high molecular weight oligomers is intended at the outlet of the oligomerization reactor for removal thereof. However, this is a complicated and operational-extensive mode of operation.

It is an object of the present invention to provide a method for the preparation of linear alpha-olefins which overcomes the drawbacks of the prior art. Especially a method shall be provided wherein deposition of heavy molecular weight oligomers can be avoided, as desired, especially in a catalyst removal section and in equipment and piping systems downstreams thereof.

Further a reactor system for carrying out such a method shall be provided.

The first object is achieved in that an outlet stream from the reactor comprising the solvent, catalyst and linear alpha-olefins is heated by at least one heating means to a temperature to allow substantially all of the linear alpha-olefins to be dissolved and/or melted in the outlet stream.

Preferably the temperature is from about 80 to about 140°C.

More preferred, the linear alpha-olefins comprise olefins having 4 to more than 20 carbon atoms.

Heating may be achieved by electrical tracing, steam tracing or a heat exchanger.

Preferably, heating is carried out in a catalyst removal section where catalyst is deactivated and removed.

In one embodiment, heating is achieved by addition of heated caustic deactivation agent within the catalyst removal section.

The temperature downstreams of the catalyst removal section can be preferably kept to be not lower than in the catalyst removal section until final disposal of undesired components of the outlet stream.

Here it is preferred that non-desired components are catalyst constituents and/or linear alpha-olefins having more than 20 carbon atoms.

More preferred, the final disposal is from a bottoms of a separation column.

Even preferred, an oligomerization is carried out utilizing a homogeneous catalyst comprising a zirconium component and an organoaluminum component.

Moreover, it is preferred that the zirconium component has the formula ZrCl₄₋ₘXₘ, wherein X=OCOR or OSO₃R' with R and R' being independently alkyl, alkene and phenyl, and wherein 0<m<4.

In one embodiment, the organoaluminum component is Al(C₂H₅)₃, Al₂Cl₃(C₂H₅)₃, AlCl(C₂H₅)₂ or a mixture thereof.

Preferably, the residence time of the alpha olefin stream from the reactor outlet to the catalyst removal section is below 2 minutes.

According to the invention is also a reactor system for the preparation of linear alpha-olefins, especially by a method according to the present invention, comprising a reactor having an outlet for discharging an outlet stream comprising solvent, catalyst and linear alpha-olefins, and heating means to heat the outlet stream from the reactor.

Preferably, the heating means is placed in a catalyst removal section connected to the reactor.

Finally, the catalyst removal section is preferably adjacent to the reactor outlet, in order to maintain the residence time of the alpha olefin stream from the reactor outlet to the catalyst removal section below 2 minutes.

Surprisingly, it was found that by provision of heating means in a reactor system for preparing linear alpha-olefins, preferably directly adjacent to the reactor, deposition of heavy molecular weight oligomers can be avoided, and therefore plugging of equipment and piping systems. In the inventive method it is important that the temperature of the outlet stream is adjusted to allow substantially all of the linear alpha-olefins, including heavy molecular weight oligomers, to be dissolved and/or melted therein.

This temperature should be maintained until final disposal of non-desired components of this outlet stream, in order to avoid plugging of equipment and piping systems downstreams the heating means. Preferably, heating is carried out in the catalyst removal section which is preferably immediately adjacent to the reactor outlet in order to deactivate the catalyst as soon as possible to avoid further oligomerization in reactor piping and equipment.

It is obvious that the inventive method is not necessarily restricted to the oligomerization of ethylene to obtain linear alpha-olefins, but may be utilized in all technologies handling high molecular weight oligomers.

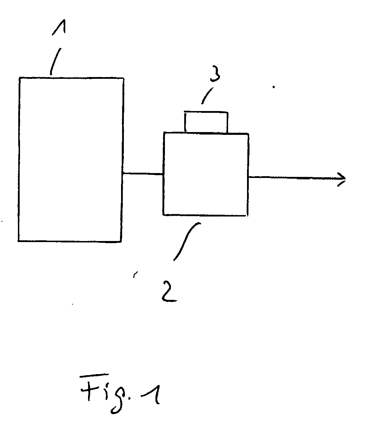

Additional features and advantages of the present invention are now illustrated in detail with reference to the accompanying drawing wherein
Figure 1 shows a schematic illustration of the inventive method for the preparation of linear alpha-olefins.

In figure 1 a reactor 1 is shown which can be utilized for the oligomerization of ethylene to prepare linear-alpha olefins. In the reactor 1 ethylene is oligomerized in the presence of solvent and catalyst, preferably at a temperature of about 60-100°C. After oligomerization (the reactor is preferably operated continuously), an outlet stream is removed from the reactor. The outlet stream comprises the solvent, catalyst and linear alpha-olefins, either with low and high molecular weight. In detail, the term "high molecular weight oligomer" is meant to comprise oligomers having such a high molecular weight that they are substantially solid at reaction temperature. The outlet stream from the reactor 1 is then preferably transferred to a catalyst removal section 2 where catalyst is deactivated and removed by addition of a catalyst deactivation agent, e.g. a solution of sodium hydroxide. It is preferred that the residence time of the outlet stream from the reactor to the catalyst removal section is as short as possible, preferably below 2 minutes. At the catalyst removal section 2, there is also provided a heating means 3 to heat the outlet stream to a temperature so that the linear alpha-olefins are substantially all dissolved and/or melted in the outlet stream. Someone skilled in the art is aware of any heating means which can be utilized for this purpose. After the catalyst removal section 2 the outlet stream can be further processed, e.g. the linear alpha-olefins may be separated from solvent and catalyst residues. Especially, the linear alpha-olefins may be separated into several fractions, e.g. into fractions having low and high molecular weight. The fraction of high molecular weight oligomers may be finally disposed, preferably from a bottoms of a separation column. It is evident for someone skilled in the art that there may be provided additional heating means downstreams of the catalyst removal section to keep the temperature of the outlet stream to be not lower than in the catalyst removal section, so that deposition of high molecular weight oligomers may be avoided in additional equipment and pipings.

## Claims

1. Method for the preparation of linear alpha-olefins by oligomerization of ethylene in a reactor in the presence of a catalyst and solvent **characterized in that** an outlet stream from the reactor comprising the solvent, catalyst and linear alpha-olefins is heated by at least one heating means to a temperature to allow substantially all of the linear alpha-olefins to be dissolved and/or melted in the outlet stream.

2. Method according to claim 1 wherein the temperature is from about 80 to about 140°C.

3. Method according to claim 1 or 2, wherein the linear alpha-olefins comprise olefins having 4 to more than 20 carbon atoms.

4. Method according to any of the preceding claims, wherein heating is achieved by electrical tracing, steam tracing or a heat exchanger.

5. Method according to any of the preceding claims, wherein heating is carried out in a catalyst removal section where catalyst is deactivated and removed.

6. Method according to claim 5, wherein heating is achieved by addition of heated caustic deactivation agent within the catalyst removal section.

7. Method according to claim 5 or 6, wherein the temperature downstreams of the catalyst removal section is kept to be not lower than in the catalyst removal section until final disposal of undesired components of the outlet stream.

8. Method according to claim 7, wherein non-desired components are catalyst constituents and/or linear alpha-olefins having more than 20 carbon atoms.

9. Method according to claim 7 or 8, wherein final disposal is from a bottoms of a separation column.

10. Method according to any of the claims 1 to 9, wherein an oligomerization is carried out utilizing a homogeneous catalyst comprising a zirconium component and an organoaluminum component.

11. Method according to any of the claims I to 10, wherein the zirconium component has the formula ZrCl₄₋ₘXₘ, wherein X=OCOR or OSO₃R' with R and R' being independently alkyl, alkene and phenyl, and wherein 0<m<4.

12. Method according to any of the claims 1 to 11, wherein the organoaluminum component is Al(C₂H₅)₃, Al₂Cl₃(C₂H₅)₃, AlCl(C₂H₅)₂ or a mixture thereof.

13. Method according to any of the claims 1 to 12 wherein the residence time of the alpha olefin stream from the reactor outlet to the catalyst removal section is below 2 minutes.

14. Reactor system for the preparation of linear alpha-olefins, especially by a method according to any of the preceding claims, comprising a reactor having an outlet for discharging an outlet stream comprising solvent, catalyst and linear alpha-olefins, and heating means to heat the outlet stream from the reactor, wherein the heating means is placed in a catalyst removal section connected to the reactor.

15. Reactor system according to claim 14, wherein the catalyst removal section is installed directly adjacent to the reactor outlet.

16. Reactor system according to claim 15 wherein the residence time of the alpha olefin stream from the reactor outlet to the catalyst removal section is below 2 minutes.

## Patentansprüche

1. Verfahren zur Herstellung von linearen alpha-Olefinen durch Oligomerisation von Ethylen in einem Reaktor in der Gegenwart eines Katalysators und von Lösungsmittel, **dadurch gekennzeichnet, dass** ein Auslassstrom aus dem Reaktor, der das Lösungsmittel, Katalysator und lineare alpha-Olefine umfasst, durch wenigstens ein Heizmittel auf eine Temperatur erwärmt wird, um es im wesentlichen allen der linearen alpha-Olefine zu erlauben, in dem Auslassstrom gelöst und/oder aufgeschmolzen zu werden.

2. Verfahren nach Anspruch 1, wobei die Temperatur von etwa 80 bis etwa 140°C beträgt.

3. Verfahren nach Anspruch 1 oder 2, wobei die linearen alpha-Olefine Olefine mit 4 bis mehr als 20 Kohlenstoffatomen umfassen.

4. Verfahren nach einem der vorangehenden Ansprüche, wobei das Erwärmen erreicht wird durch elektrische Beheizung, Dampfbeheizung oder einen Wärmeaustauscher.

5. Verfahren nach einem der vorangehenden Ansprüche, wobei das Erwärmen durchgeführt wird in einem Katalysatorentfernungsabschnitt, wo der Katalysator deaktiviert und entfernt wird.

6. Verfahren nach Anspruch 5, wobei das Erwärmen erreicht wird durch Zugabe eines erwärmten alkalischen Deaktivierungsagens innerhalb des Katalysatorentfernungsabschnitts.

7. Verfahren nach Anspruch 5 oder 6, wobei die Temperatur stromabwärts des Katalysatorentfernungsabschnitts so gehalten wird, um nicht geringer zu sein als in dem Katalysatorentfernungsabschnitt, bis zur endgültigen Abgabe unerwünschter Komponenten des Auslassstroms.

8. Verfahren nach Anspruch 7, wobei unerwünschte Komponenten Katalysatorbestandteile und/oder lineare alpha-Olefine mit mehr als 20 Kohlenstoffatomen sind.

9. Verfahren nach Anspruch 7 oder 8, wobei eine endgültige Abgabe von einem Bodenprodukt einer Trennsäule erfolgt.

10. Verfahren nach einem der Ansprüche 1 bis 9, wobei eine Oligomerisation durchgeführt wird unter Einsatz eines homogenen Katalysators umfassend eine Zirkoniumkomponente und eine Organoaluminiumkomponente.

11. Verfahren nach einem der Ansprüche 1 bis 10, wobei die Zirkoniumkomponente die Formel ZrCl₄₋ₘXₘ aufweist, wobei X = OCOR oder OSO₃R' ist, wobei R und R' unabhängig Alkyl, Alken und Phenyl sind, und wobei 0 < m < 4 ist.

12. Verfahren nach einem der Ansprüche 1 bis 11, wobei die Organoaluminiumkomponente Al(C₂H₅)₃, Al₂Cl₃(C₂H₅)₃, AlCl(C₂H₅)₂ oder eine Mischung davon ist.

13. Verfahren nach einem der Ansprüche 1 bis 12, wobei die Verweilzeit des alpha-Olefinstroms von dem Reaktorauslass zum Katalysatorentfernungsabschnitt unter 2 Minuten beträgt.

14. Reaktorsystem für die Herstellung von linearen alpha-Olefinen, insbesondere durch ein Verfahren gemäß einem der vorangehenden Ansprüche, umfassend einen Reaktor mit einem Auslass zur Abgabe eines Auslassstroms umfassend Lösungsmittel, Katalysator und linare alpha-Olefine, und ein Heizmittel, um den Auslassstrom aus dem Reaktor zu erwärmen, wobei das Heizmittel in einem Katalysatorentfernungsabschnitt, der mit dem Reaktor verbunden ist, angeordnet ist.

15. Reaktorsystem nach Anspruch 14, wobei der Katalysatorentfernungsabschnitt direkt angrenzend an den Reaktorauslass installiert ist.

16. Reaktorsystem nach Anspruch 15, wobei die Verweilzeit des alpha-Olefinstroms aus dem Reaktorauslass zum Katalysatorentfernungsabschnitt unter 2 Minuten beträgt.

## Revendications

1. Procédé de préparation d'alpha-oléfines linéaires par oligomérisation d'éthylène dans un réacteur en présence d'un catalyseur et d'un solvant, **caractérisé en ce qu'**un flux de sortie du réacteur comprenant le solvant, le catalyseur et les alpha-oléfines linéaires est chauffé par au moins un moyen de chauffage à une température devant permettre la dissolution et/ou la fusion de sensiblement toutes les alpha-oléfines linéaires dans le flux de sortie.

2. Procédé selon la revendication 1, dans lequel la température est comprise entre environ 80 et environ 140°C.

3. Procédé selon la revendication 1 ou 2, dans lequel les alpha-oléfines linéaires comprennent des oléfines ayant de 4 à plus de 20 atomes de carbone.

4. Procédé selon l'une quelconque des revendications précédentes, dans lequel le chauffage est réalisé par traçage électrique, traçage à la vapeur ou par un échangeur de chaleur.

5. Procédé selon l'une quelconque des revendications précédentes, dans lequel le chauffage est réalisé dans une section d'élimination de catalyseur où le catalyseur est désactivé et éliminé.

6. Procédé selon la revendication 5, dans lequel le chauffage est réalisé par addition d'agent de désactivation caustique chauffé à l'intérieur de la section d'élimination de catalyseur.

7. Procédé selon la revendication 5 ou 6, dans lequel la température en aval de la section d'élimination de catalyseur est maintenue non inférieure à la température dans la section d'élimination de catalyseur jusqu'à l'élimination finale de composants indésirés du flux de sortie.

8. Procédé selon la revendication 7, dans lequel les composants indésirés sont des constituants de catalyseur et/ou des alpha-oléfines linéaires ayant plus de 20 atomes de carbone.

9. Procédé selon la revendication 7 ou 8, dans lequel l'élimination finale s'opère à partir d'un fond d'une colonne de séparation.

10. Procédé selon l'une quelconque des revendications 1 à 9, dans lequel une oligomérisation est réalisée en utilisant un catalyseur homogène comprenant un composant zirconium et un composant organoaluminium.

11. Procédé selon l'une quelconque des revendications 1 à 10, dans lequel le composant zirconium est de formule ZrCl₄₋ₘXₘ, où X=OCOR ou OSO₃R', R et R' étant indépendamment de l'alkyle, de l'alkène et du phényle, et où 0<m<4.

12. Procédé selon l'une quelconque des revendications 1 à 11, dans lequel le composant organoaluminium est du Al(C₂H₅)₃, Al₂Cl₃(C₂H₅)₃, AlCl(C₂H₅) ou leur mélange.

13. Procédé selon l'une quelconque des revendications 1 à 12, dans lequel le temps de séjour du flux d'alpha-oléfines de la sortie du réacteur à la section d'élimination de catalyseur est inférieur à 2 minutes.

14. Système réacteur pour la préparation d'alpha-oléfines linéaires, en particulier au moyen d'un procédé selon l'une quelconque des revendications précédentes, comprenant un réacteur présentant une sortie pour évacuer un flux de sortie comprenant un solvant, un catalyseur et des alpha-oléfines linéaires, et un moyen de chauffage pour chauffer le flux de sortie du réacteur, le moyen de chauffage étant placé dans une section d'élimination de catalyseur raccordée au réacteur.

15. Système réacteur selon la revendication 14, dans lequel la section d'élimination de catalyseur est installée de manière directement adjacente à la sortie du réacteur.

16. Système réacteur selon la revendication 15, dans lequel le temps de séjour du flux d'alpha-oléfines de la sortie du réacteur à la section d'élimination de catalyseur est inférieur à 2 minutes.
